(19) Europäisches Patentamt
European Patent Office
Office européen des brevets

(11) Veröffentlichungsnummer: **0 175 233**
**A2**

(12) # EUROPÄISCHE PATENTANMELDUNG

(21) Anmeldenummer: **85111269.8**

(22) Anmeldetag: **06.09.85**

(51) Int. Cl.⁴: **C 07 D 249/08**, C 07 D 233/61, A 01 N 43/653, A 01 N 43/50 // C07C149/34

(30) Priorität: **18.09.84 DE 3434158**

(43) Veröffentlichungstag der Anmeldung: **26.03.86 Patentblatt 86/13**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Elbe, Hans-Ludwig, Dr., Dasnöckel 59, D-5600 Wuppertal 11 (DE)**
Erfinder: **Büchel, Karl Heinz, Prof., Dr., Dabringhausener Strasse 42, D-5093 Burscheid (DE)**
Erfinder: **Hänssler, Gerd, Dr., Heymannstrasse 40, D-5090 Leverkussen 1 (DE)**

(54) **Substituierte 1,1-Bis-azolyl-butan-2-one und -ole.**

(57) Neue substituierte 1,1-Bis-azolyl-butan-2-one und -ole der Formel

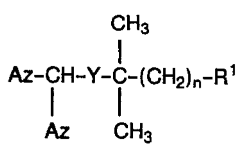

(I)

in welcher
Az für 1,2,4-Triazol-1-yl oder Imidazol-1-yl steht,
$R^1$ für gegebenenfalls substituiertes Aryl oder die Gruppierung $-XR^2$ steht, wobei
$R^2$ für Alkyl, Halogenalkyl, gegebenenfalls substituiertes Aryl oder gegebenenfalls substituiertes Aralkyl steht und
X für Sauerstoff oder Schwefel steht,
n für die Zahlen 0, 1 oder 2 steht und
Y für die CO- oder CH(OH)-Gruppe steht,
sowie deren Säureadditions-Salze und Metallsalz-Komplexe, ein Verfahren zur Herstellung der neuen Stoffe und deren Verwendung als Fungizide.

BAYER AKTIENGESELLSCHAFT
Konzernverwaltung RP
Patentabteilung

5090 Leverkusen, Bayerwerk

Slr/bo/c
Ia

## Substituierte 1,1-Bis-azolyl-butan-2-one und -ole

Die vorliegende Erfindung betrifft neue substituierte 1,1-Bis-azolyl-butan-2-one und -ole, ein Verfahren zu ihrer Herstellung sowie ihre Verwendung als Fungizide.

Es ist bereits bekannt geworden, daß bestimmte 1-Azolyl-butan-2-one und -ole, wie beispielsweise 4-Chlorphenyl-3,3-dimethyl-1-(1,2,4-triazol-1-yl)-butan-2-on und -ol, allgemein gute fungizide Wirksamkeit besitzen (vergleiche EP-A 0 055 833 /Le A 20 762/ und EP-A 0 054 865 /Le A 20 763/). Die Wirkung dieser Verbindungen ist jedoch in bestimmten Indikationsbereichen, insbesondere bei niedrigen Aufwandmengen und -konzentrationen, nicht immer voll befriedigend.

Es wurden nun neue substituierte 1,1-Bis-azolyl-butan-2-one und -ole der allgemeinen Formel

$$Az-\underset{\displaystyle Az}{CH}-Y-\overset{\displaystyle CH_3}{\underset{\displaystyle CH_3}{C}}-(CH_2)_n-R^1 \qquad (I)$$

in welcher

Az für 1,2,4-Triazol-1-yl oder Imidazol-1-yl steht,

$R^1$ für gegebenenfalls substituiertes Aryl oder die Gruppierung $-XR^2$ steht, wobei

$R^2$ für Alkyl, Halogenalkyl, gegebenenfalls substituiertes Aryl oder gegebenenfalls substituiertes Aralkyl steht und

X für Sauerstoff oder Schwefel steht,

n für die Zahlen 0, 1 oder 2 steht und

Y für die CO- oder CH(OH)-Gruppe steht,

sowie deren Säureadditions-Salze und Metallsalz-Komplexe gefunden.

Weiterhin wurde gefunden, daß man die substituierten 1,1-Bis-azolyl-butan-2-one und -ole der Formel (I) sowie deren Säureadditions-Salze und Metallsalz-Komplexe erhält, wenn man Dihalogenmethylketone der Formel

$$\begin{array}{lll} & & CH_3 \\ & & \vert \\ Hal- & CH-CO- & C-(CH_2)_n-R^1 \\ & \vert & \vert \\ & Hal & CH_3 \end{array} \qquad (II)$$

in welcher

Hal für Halogen, insbesondere Chlor oder Brom steht und

$R^1$ und n die oben angegebene Bedeutung haben,

mit Azolen der Formel

H - Az (III)

in welcher

Az die oben angegebene Bedeutung hat,

in Gegenwart eines Verdünnungsmittels und in Gegenwart eines Säurebindemittels umsetzt, und gegebenenfalls noch die so erhaltenen Keto-Derivate der Formel (I) nach bekannten Methoden in üblicher Weise reduziert.

An die so erhaltenen Verbindungen der Formel (I) kann gegebenenfalls anschließend eine Säure oder ein Metallsalz addiert werden.

Schließlich wurde gefunden, daß die neuen substituierten 1,1-Bis-azolyl-butan-2-one und -ole der Formel (I) sowie deren Säureadditions-Salze und Metallsalz-Komplexe starke fungizide Eigenschaften aufweisen.

Außerdem sind die neuen substituierten 1,1-Bis-azolyl-butan-2-one und -ole der Formel (I) interessante Zwischenprodukte zur Herstellung von weiteren Pflanzenschutzmitteln. Bei den Keto-Derivaten kann die Ketogruppe zu einer -CH(OH)-Gruppe bzw. einer CR(OH)-Gruppe reduziert werden. Ferner können durch entsprechende Umsetzung funktionelle Derivate der Ketogruppe erhalten werden, wie z.B. Oxime und Oximether, Hydrazone und Ketale. Die Carbinol-Derivate können an der Hydroxygruppe in üblicher Weise in die entsprechenden Ether überführt werden. Weiterhin können durch Umsetzung mit z.B. Acylhalogeniden oder Carbamoylchloriden in prinzipiell bekannter Weise Acyl- oder Carbamoyl-Derivate der Verbindungen der Formel (I) erhalten werden.

Überraschenderweise besitzen die erfindungsgemäßen Verbindungen eine bessere fungizide Wirkung als die aus dem Stand der Technik bekannten 1-Azolyl-butan-2-one und-ole, wie z.B. das 4-Chlorphenyl-3,3-dimethyl-1-(1,2,4-triazol-1-yl)-butan-2-on und -ol, welches chemisch und biologisch naheliegende Verbindungen sind. Die erfindungsgemäßen Wirkstoffe stellen somit eine Bereicherung der Technik dar.

Die erfindungsgemäßen substituierten 1,1-Bis-azolylbutan-2-one und -ole sind durch die Formel (I) allgemein definiert. In dieser Formel stehen vorzugsweise

Az für 1,2,4-Triazol-1-yl oder Imidazol-1-yl;

$R^1$ für gegebenenfalls einfach bis dreifach, gleich oder verschieden substituiertes Phenyl, wobei als Substituenten Halogen, Alkyl mit 1 bis 4 Kohlenstoffatomen, Alkoxy und Alkylthio mit jeweils 1 oder 2 Kohlenstoffatomen, Cyclohexyl, Dialkylamino mit 1 bis 4 Kohlenstoffatomen in jedem Alkylteil, Halogenalkyl, Halogenalkoxy und Halogenalkylthio mit jeweils 1 bis 2 Kohlenstoffatomen und 1 bis 5 gleichen oder verschiedenen Halogenatomen, Nitro, Cyano, Alkoxycarbonyl mit 1 bis 4 Kohlenstoffatomen im Alkylteil, jeweils gegebenenfalls durch Halogen substituiertes Phenyl und Phenoxy, Hydroximinoalkyl mit 1 bis 4 Kohlenstoffatomen und Alkoximinoalkyl mit 1 bis 4 Kohlenstoffatomen in jedem Alkylteil zu nennen sind, ferner für die Gruppierung $-XR^2$, wobei

X für Sauerstoff oder Schwefel steht und

$R^2$ für geradkettiges oder verzweigtes Alkyl mit 1 bis 6 Kohlenstoffatomen, Halogenalkyl mit 1 bis 4 Kohlenstoffatomen und 1 bis 5 gleichen oder verschiedenen Halogenatomen sowie für jeweils gegebenenfalls einfach bis dreifach, gleich oder verschieden substituiertes Phenyl oder Phenylalkyl mit 1 bis 2 Kohlenstoffatomen im Alkylteil steht,
wobei als Phenylsubstituenten die bei $R^1$ bereits genannten Substituenten in Frage kommen;

n für die Zahlen 0, 1 oder 2 und

Y für die CO- oder CH(OH)-Gruppe.

Besonders bevorzugt sind diejenigen Verbindungen der Formel (I), in denen

Az für 1,2,4-Triazol-1-yl oder Imidazol-1-yl steht;

$R^1$ für einfach oder zweifach, gleich oder verschieden substituiertes Phenyl steht, wobei als Substituenten genannt seien: Fluor, Chlor, Brom, Methyl, Ethyl, Isopropyl, tert.-Butyl, Methoxy, Methylthio, Cyclohexyl, Dimethylamino, Trifluormethyl, Trifluormethoxy, Trifluormethylthio, Nitro, Cyano, jeweils gegebenenfalls durch Fluor und Chlor substituiertes Phenyl und Phenoxy, Methoxycarbonyl, Ethoxycarbonyl, Hydroximinomethyl, 1-Hydroximinoethyl, Methoximinomethyl sowie 1-Methoximinoethyl; sowie für die Gruppierung $-XR^2$ steht, wobei

X für Sauerstoff oder Schwefel steht und

$R^2$ für Methyl, Ethyl, n-Propyl, Isopropyl, n-Butyl, Isobutyl oder jeweils gegebenenfalls einfach bis zweifach, gleich oder verschieden substituiertes Phenyl und Benzyl steht, wobei als Substituenten die bei $R^1$ bereits genannten Phenylsubstituenten in Frage kommen;

n für die Zahlen 0, 1 oder 2 steht und

Y für die CO- oder CH(OH)-Gruppe steht.

Bevorzugte erfindungsgemäße Verbindungen sind auch Additionsprodukte aus Säuren und denjenigen substituierten 1,1-Bis-azolyl-butan-2-onen und -olen der Formel (I), in denen $R^1$, $R^2$, X, Y, Az und der Index n die Bedeutungen haben, die bereits vorzugsweise für diese Reste und den Index genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen substituierten 1,1-Bis-azolyl-butan-2-onen und -olen der Formel (I), in denen $R^1$, $R^2$, X, Y, Az und der Index n die Bedeutungen haben, die bereits vorzugsweise für diese Reste und den Index genannt wurden.

Hierbei sind Salze des Kupfers, Zinks, Mangans, Magnesiums, Zinns, Eisens und des Nickels besonders bevorzugt. Als Anionen dieser Salze kommen solche in

0175233

Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man beispielsweise 1,1-Dibrom-3-(4-chlorphenoxy)-3-methyl-2-butanon und 1,2,4-Triazol als Ausgangsstoffe, so kann der Reaktionsablauf des erfindungsgemäßen Verfahrens durch das folgende Formelschema wiedergegeben werden:

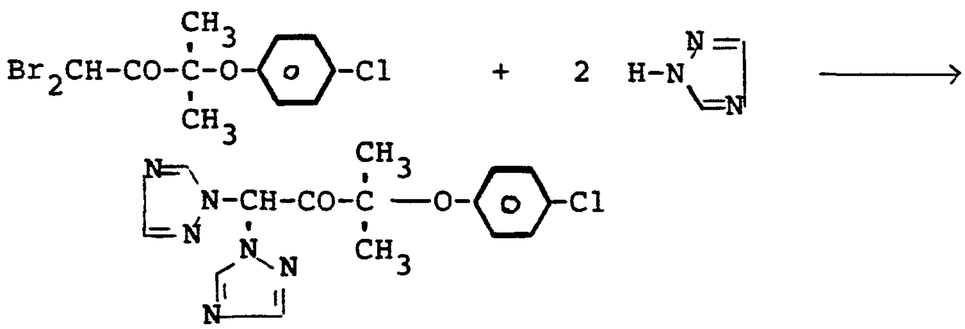

Verwendet man beispielsweise 3-(4-Chlorphenoxy)-1,1-di-(1,2,4-triazol-1-yl)-3-methyl-2-butanon und Natriumborhydrid als Ausgangsstoffe, so kann der Reaktionsablauf der erfindungsgemäßen Reduktion durch das folgende Formelschema wiedergegeben werden:

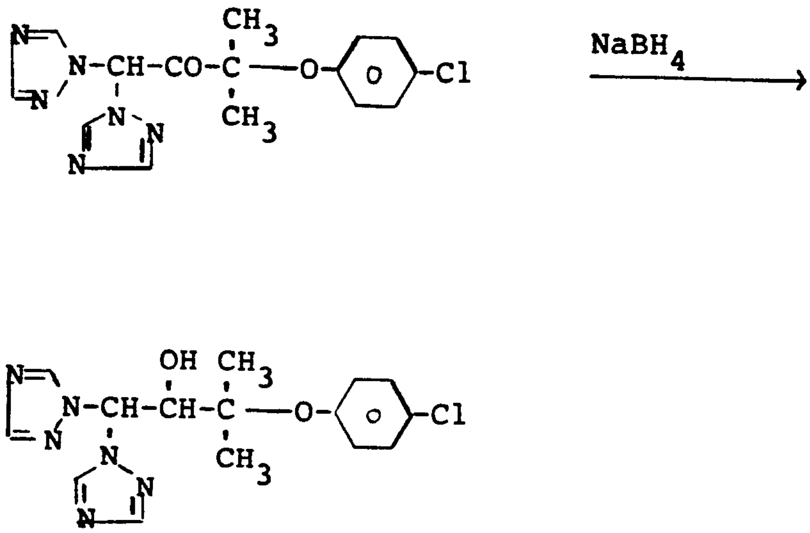


Die als Ausgangsstoffe für das erfindungsgemäße Verfahren zu verwendenden Dihalogenmethyl-ketone sind durch die Formel (II) allgemein definiert. In dieser Formel stehen $R^1$ und der Index n vorzugsweise für diejenigen Bedeutungen, die bereits im Zusammenhang mit der Beschreibung der erfindungsgemäßen Stoffe der Formel (I) vorzugsweise für diesen Substituenten bzw. Index genannt wurden. Hal steht vorzugsweise für Brom oder Chlor.

Die Dihalogenmethyl-ketone der Formel (II) sind noch nicht bekannt. Sie können in allgemein üblicher Art und Weise erhalten werden, indem man entsprechende Ketone der Formel (IV)

$$CH_3 - CO - \underset{CH_3}{\overset{CH_3}{C}} - (CH_2)_n - R^1 \qquad (IV)$$

in welcher

$R^1$ und n die oben angegebene Bedeutung haben,

mit Chlor oder Brom in Gegenwart eines inerten organischen Lösungsmittels, wie beispielsweise Ether, chlorierten oder nicht chlorierten Kohlenwasserstoffen, bei Raumtemperatur umsetzt; oder mit üblichen Chlorierungsmitteln, wie beispielsweise Sulfurylchlorid, bei 20 bis 60°C umsetzt.

Die Ketone der Formel (IV) sind bekannt (vergleiche z.B. EP-A 0 054 865 /Le A 20 763/ und EP-A 0 084 834 /Le A 21 425/).

Die außerdem für das erfindungsgemäße Verfahren als Ausgangsstoffe zu verwendenden Azole der Formel (III) sind allgemein bekannte Verbindungen der organischen Chemie.

Für das erfindungsgemäße Verfahren kommen als Verdünnungsmittel inerte organische Lösungsmittel in Frage. Hierzu gehören vorzugsweise Ketone, wie Diethylketon und insbesondere Aceton und Methylethylketon; Nitrile, wie Propionitril, insbesondere Acetonitril; Alkohole wie Ethanol oder Isopropanol; Ether, wie Tetrahydrofuran oder Dioxan; aromatische Kohlenwasserstoffe, wie Toluol, Benzol oder Chlorbenzol; Formamide, wie insbesondere Dimethylformamid; und halogenierte Kohlenwasserstoffe.

Das erfindungsgemäße Verfahren wird in Gegenwart eines Säurebindemittels vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat oder wie niedere tertiäre Alkylamine, Cycloalkylamine oder Aralkylamine, beispielsweise Triethylamin, N,N-Dimethyl-cyclohexylamin, Dicyclohexylamin, N,N-Dimethylbenzylamin, weiterhin Pyridin und Diazabicyclooctan. Vorzugsweise verwendet man einen entsprechenden Überschuß an Azol.

Die Reaktionstemperaturen können bei dem erfindungsgemäßen Verfahren in einem größeren Bereich variiert werden. Im allgemeinen arbeitet man zwischen etwa 20 bis etwa 150°C, vorzugsweise bei 60 bis 120°C. Bei Anwesenheit eines Lösungsmittels wird zweckmäßigerweise beim Siedepunkt des jeweiligen Lösungsmittels gearbeitet.

Bei der Durchführung des erfindungsgemäßen Verfahrens setzt man auf 1 Mol der Verbindungen der Formel (II) vorzugsweise 2 bis 6 Mol Azol und 2 bis 6 Mol Säurebinder ein. Zur Isolierung der Verbindungen der Formel (I) wird das Lösungsmittel abdestilliert und der Rückstand in üblicher Art und Weise aufgearbeitet.

Die erfindungsgemäße Reduktion erfolgt in üblicher Weise, wie z.B. durch Umsetzung mit komplexen Hydriden, gegebenenfalls in Gegenwart eines Verdünnungsmittels; oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel in Frage. Hierzu gehören vorzugsweise Alkohole, wie Methanol, Ethanol, Butanol, Isopropanol und Ether, wie Diethylether oder Tetrahydrofuran.

Die Reaktion wird im allgemeinen bei 0 bis 30°C, vorzugsweise bei 0 bis 20°C durchgeführt. Hierzu setzt man auf 1 Mol Keto-Derivat der Formel (I) etwa 1 Reduktionsäquivalent eines komplexen Hydrids, wie Natriumhydrid oder Lithiumalanat, ein. Zur Isolierung der reduzierten Verbindungen der Formel (I) wird der Rückstand mit verdünnter Salzsäure neutral gestellt, im Vakuum eingeengt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemäße Umsetzung bevorzugt Alkohole, wie Isopropanol oder inerte Kohlenwasserstoffe, wie Benzol in Frage. Die Reaktionstemperaturen können wiederum in einem größeren Bereich variiert werden; im allgemeinen arbeitet man zwischen 20 und 120°C, vorzugsweise bei 50 bis 100°C. Zur Durchführung der Reaktion setzt man auf 1 Mol Keto-Derivat der Formel (I) etwa 1 bis 2 Mol Aluminiumisopropylat ein. Zur Isolierung der reduzierten Verbindungen der Formel (I) wird das überschüssige Lösungsmittel durch Destillation im Vakuum entfernt

und die entstandene Aluminiumverbindung mit verdünnter Schwefelsäure oder Natronlauge zersetzt. Die weitere Aufarbeitung erfolgt in üblicher Weise.

Zur Herstellung von Säureadditions-Salzen der substituierten 1,1-Bis-azolyl-butan-2-one und -ole der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen von substituierten 1,1-Bis-azolyl-butan-2-onen und -olen der Formel (I) kommen vorzugsweise Salze von denjenigen Anionen und Kationen in Betracht, die bereits im Zusammenhang mit der Beschreibung der erfindungsgemäßen Metallsalz-Komplexe als bevorzugt genannt wurden.

Die Metallsalz-Komplexe der Verbindungen der Formel (I) können in einfacher Weise nach üblichen Verfahren

erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren, Isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Reiskrankheiten, wie Pyricularia oryzae, von Apfelschorf und Bohnenrost sowie von Mehltau, Septoria nodorum und Pyrenophora teres an Getreide eingesetzt werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut,

vorzugsweise 0,1 bis 10 g, benötigt. Bei der Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1

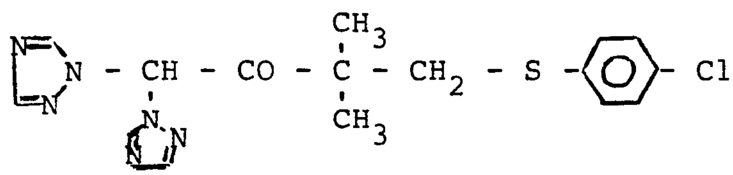

79,3 g (0,2 Mol) 4-(4-Chlorphenylthio)-1,1-dibrom-3,3-dimethyl-2-butanon werden zu 41,4 g (0,6 Mol) 1,2,4-Triazol und 166 g Kaliumcarbonat in 700 ml Aceton gegeben. Nach beendeter Zugabe hält man das Reaktionsgemisch während 10 Stunden im Sieden und läßt unter Rückfluß rühren. Zur Aufarbeitung läßt man abkühlen und saugt über eine Nutsche ab. Die Mutterlauge wird eingeengt, der Rückstand in Methylenchlorid aufgenommen, zweimal mit Wasser gewaschen, über Natriumsulfat getrocknet und eingeengt. Der Rückstand wird mit Ether verrührt, eingeengt und getrocknet. Man erhält 45 g (59,8 % der Theorie) 4-(4-Chlorphenylthio)-3,3-dimethyl-1,1-di-(1,2,4-triazol-1-yl)-2-butanon vom Schmelzpunkt 122°C.

Herstellung des Ausgangsproduktes

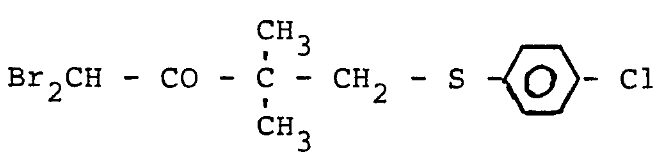

48,5 g (0,2 Mol) 1-(4-Chlorphenylthio)-2,2-dimethyl-3-butanon in 200 ml Chloroform werden bei Raumtemperatur tropfenweise mit 20,3 ml (0,4 Mol) Brom versetzt. Nach beendeter Zugabe läßt man 30 Minuten bei Raumtemperatur nachrühren. Anschließend wird eingeengt. Man erhält 79,3 g (99 % der Theorie) 4-(4-Chlorphenylthio)-1,1-dibrom-3,3-dimethyl-2-butanon vom Brechungsindex $n_D^{20}$ = 1,6083.

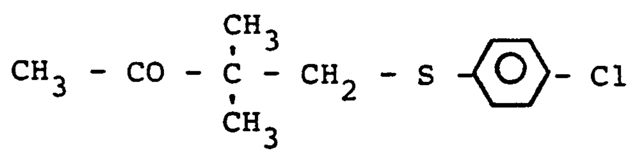

134,5 g (1 Mol) 4-Chlorpinakolin werden mit 216 g (1,5 Mol) 4-Chlorthiophenol und 210 g (1,52 Mol) Kaliumcarbonat in 500 ml Dimethylformamid 15 Stunden bei 150°C und einem Druck von 2 bis 4 bar gerührt. Man läßt auf Raumtemperatur abkühlen, verrührt mit 10 l Wasser und extrahiert mit Ether. Die Etherphase wird über Natriumsulfat getrocknet, eingeengt und der Rückstand im Vakuum destilliert. Man erhält 151 g (62 % der Theorie) 1-(4-Chlorphenylmercapto)-2,2-dimethylbutan-3-on vom Siedepunkt 146°C/0,5 mbar.

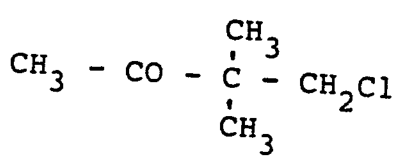

11,6 g (0,1 Mol) 2,2-Dimethyl-1-hydroxy-butan-3-on (Herstellung vgl. Beispiel 3) werden bei 50 bis 60°C

(Eiskühlung) zu 20,5 g (0,1 Mol) N,N-Diethyl-1,2,2-trichlorvinyl-amin getropft. Nach zweistündigem Rühren bei 60°C wird im Wasserstrahlvakuum destilliert. Man erhält 8,1 g (60 % der Theorie) 4-Chlorpinakolin vom Schmelzpunkt 60 - 62°C/12 mbar.

Beispiel 2

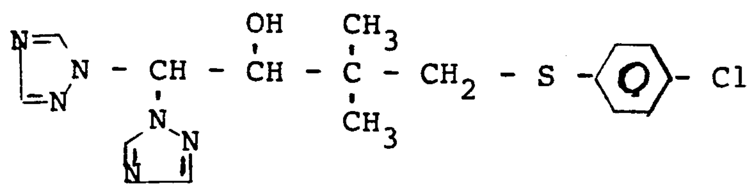

Zu 15 g (0,04 Mol) 4-(4-Chlorphenylthio)-3,3-dimethyl-1,1-di-(1,2,4-triazol-1-yl)-2-butanon (Beispiel 1) in 200 ml Methanol gibt man 0,5 g (0,013 Mol) Natriumborhydrid in 5 ml Wasser. Man läßt das Reaktionsgemisch 2 Stunden bei Raumtemperatur nachrühren. Anschließend wird mit verdünnter Salzsäure auf einen pH-Wert von 5 bis 6 eingestellt und eingeengt. Der Rückstand wird in Methylenchlorid aufgenommen, über Natriumsulfat getrocknet und eingeengt. Man erhält 15 g (99,7 % der Theorie) 4-(4-Chlorphenylthio)-3,3-dimethyl-1,1-di-(1,2,4-triazol-1-yl)-2-butanol als glasartiges Produkt.

In entsprechender Weise und gemäß dem angegebenen Verfahren können die folgenden Verbindungen der allgemeinen Formel (I)

$$Az - \underset{\substack{| \\ Az}}{CH} - Y - \overset{\substack{CH_3 \\ |}}{\underset{\substack{| \\ CH_3}}{C}} - (CH_2)_n - R^1 \qquad (I)$$

erhalten werden:

| Bsp.Nr. | Az | Y | n | $R^1$ | Schmelzp.(°C bzw. $n_D^{20}$ |
|---|---|---|---|---|---|
| 3 | 1,2,4-Triazol-1-yl | CO | 1 | 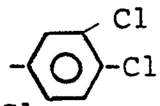 | 104 |
| 4 | 1,2,4-Triazol-1-yl | CO | 1 | 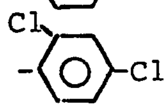 | 114 |
| 5 | 1,2,4-Triazol-1-yl | CO | 1 | 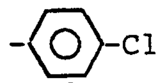 | 96 |
| 6 | 1,2,4-Triazol-1-yl | CO | 1 | 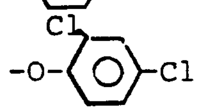 | 104-06 |
| 7 | 1,2,4-Triazol-1-yl | CO | 1 | 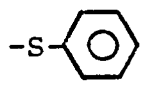 | 102-06 |
| 8 | 1,2,4-Triazol-1-yl | CO | 1 | 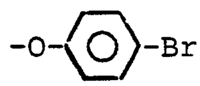 | 144 |
| 9 | 1,2,4-Triazol-1-yl | CH(OH) | 1 | 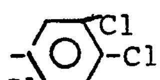 | 65-70 |
| 10 | 1,2,4-Triazol-1-yl | CH(OH) | 1 | 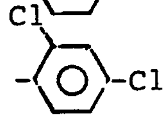 | 70-75 |
| 11 | 1,2,4-Triazol-1-yl | CH(OH) | 1 | 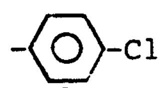 | 1,5580 |
| 12 | 1,2,4-Triazol-1-yl | CH(OH) | 1 | 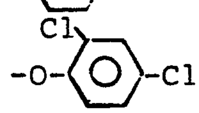 | 1,5490 |
| 13 | 1,2,4-Triazol-1-yl | CH(OH) | 1 | 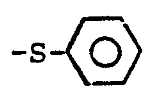 | 1,5520 |
| 14 | 1,2,4-Triazol-1-yl | CH(OH) | 1 | 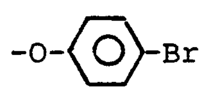 | 65-70 |

| Bsp.Nr. | Az | Y | n | $R^1$ | Schmelzp.(°C) bzw. $n_D^{20}$ |
|---|---|---|---|---|---|
| 15 | Imidazol-1-yl | CO | 1 | 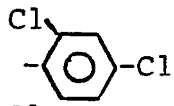 | 40 |
| 16 | Imidazol-1-yl | CH(OH) | 1 | 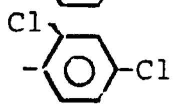 | 100 |
| 17 | 1,2,4-Triazol-1-yl | CO | 1 | 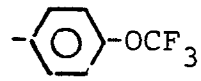 | 88 |
| 18 | Imidazol-1-yl | CO | 1 | 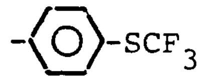 | 1,5199 |
| 19 | 1,2,4-Triazol-1-yl | CO | 1 | 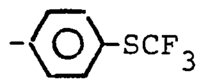 | |
| 20 | 1,2,4-Triazol-1-yl | CH(OH) | 1 | 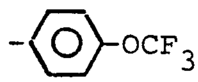 | |
| 21 | 1,2,4-Triazol-1-yl | CH(OH) | 1 | 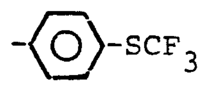 | |
| 22 | Imidazol-1-yl | CO | 1 | 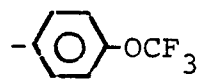 | |
| 23 | Imidazol-1-yl | CH(OH) | 1 | 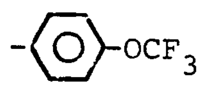 | |
| 24 | Imidazol-1-yl | CH(OH) | 1 | 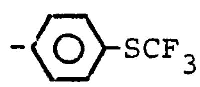 | |

Verwendungsbeispiele

In dem nachfolgenden Beispiel werden die nachstehend angegebenen Verbindungen als Vergleichssubstanzen eingesetzt:

(A) 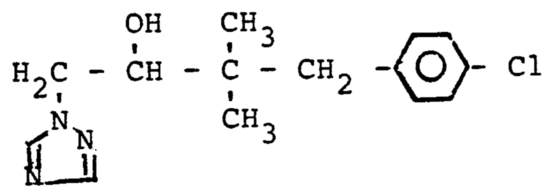

(bekannt aus EP-A 0 055 833)

(B) 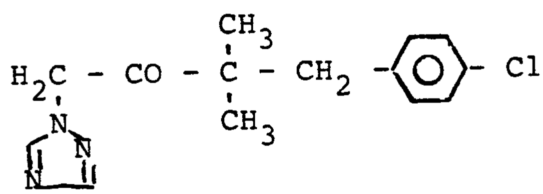

(bekannt aus EP-A 0 054 865)

Beispiel A

Pyricularia-Test (Reis) / protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber den aus dem Stand der Technik bekannten Verbindungen (A) und (B) zeigen in diesem Test z.B. die Verbindungen gemäß folgender Herstellungsbeispiele: 9, 10, 12, 5 und 6.

Patentansprüche

1) Substituierte 1,1-Bis-azolyl-butan-2-one und -ole der allgemeinen Formel

$$\begin{array}{c} \phantom{Az-CH-Y-}CH_3 \\ Az-\underset{\displaystyle Az}{CH}-Y-\underset{\displaystyle CH_3}{\overset{|}{\underset{|}{C}}}-(CH_2)_n-R^1 \end{array} \qquad (I)$$

in welcher

Az für 1,2,4-Triazol-1-yl oder Imidazol-1-yl steht,

$R^1$ für gegebenenfalls substituiertes Aryl oder die Gruppierung $-XR^2$ steht, wobei

$R^2$ für Alkyl, Halogenalkyl, gegebenenfalls substituiertes Aryl oder gegebenenfalls substituiertes Aralkyl steht und

X für Sauerstoff oder Schwefel steht,

n für die Zahlen 0, 1 oder 2 steht und

Y für die CO- oder CH(OH)-Gruppe steht,

sowie deren Säureadditions-Salze und Metallsalz-Komplexe.

2) Verbindungen der Formel (I) in Anspruch 1, wobei

Az für 1,2,4-Triazol-1-yl oder Imidazol-1-yl steht;

$R^1$ für gegebenenfalls einfach bis dreifach, gleich oder verschieden substituiertes Phenyl steht, wobei als Substituenten Halogen, Alkyl mit 1 bis 4 Kohlenstoffatomen, Alkoxy und Alkylthio mit jeweils 1 oder 2 Kohlenstoffatomen, Cyclohexyl, Dialkylamino mit 1 bis 4 Kohlenstoffatomen in jedem Alkylteil, Halogenalkyl, Halogenalkoxy und Halogenalkylthio mit jeweils 1 bis 2 Kohlenstoffatomen und 1 bis 5 gleichen oder verschiedenen Halogenatomen, Nitro, Cyano, Alkoxycarbonyl mit 1 bis 4 Kohlenstoffatomen im Alkylteil, jeweils gegebenenfalls durch Halogen substituiertes Phenyl und Phenoxy, Hydroximinoalkyl mit 1 bis 4 Kohlenstoffatomen und Alkoximinoalkyl mit 1 bis 4 Kohlenstoffatomen in jedem Alkylteil zu nennen sind, ferner für die Gruppierung $-XR^2$ steht, wobei

X für Sauerstoff oder Schwefel steht und

$R^2$ für geradkettiges oder verzweigtes Alkyl mit 1 bis 6 Kohlenstoffatomen, Halogenalkyl mit 1 bis 4 Kohlenstoffatomen und 1 bis 5 gleichen oder verschiedenen Halogenatomen steht,

sowie für jeweils gegebenenfalls einfach bis dreifach, gleich oder verschieden substituiertes Phenyl oder Phenylalkyl mit 1 bis 2 Kohlenstoffatomen im Alkylteil steht, wobei als Phenylsubstituenten die bei $R^1$ bereits genannten Substituenten zu nennen sind;

n für die Zahlen 0, 1 oder 2 steht, und

Y für die CO- oder CH(OH)-Gruppe steht.

3) Verfahren zur Herstellung von substituierten 1,1-Bis-azolyl-butan-2-onen und -olen der allgemeinen Formel

$$Az-\underset{\displaystyle Az}{CH}-Y-\underset{\displaystyle CH_3}{\overset{\displaystyle CH_3}{C}}-(CH_2)_n-R^1 \qquad (I)$$

in welcher

Az für 1,2,4-Triazol-1-yl oder Imidazol-1-yl steht,

$R^1$ für gegebenenfalls substituiertes Aryl oder die Gruppierung $-XR^2$ steht, wobei

$R^2$ für Alkyl, Halogenalkyl, gegebenenfalls substituiertes Aryl oder gegebenenfalls substituiertes Aralkyl steht und

X für Sauerstoff oder Schwefel steht,

n für die Zahlen 0, 1 oder 2 steht und

Y für die CO- oder CH(OH)-Gruppe steht,

dadurch gekennzeichnet, daß man Dihalogenmethylketone der Formel

$$\text{Hal-}\underset{\text{Hal}}{\text{CH}}\text{-CO-}\overset{CH_3}{\underset{CH_3}{\text{C}}}\text{-}(CH_2)_n\text{-}R^1 \qquad \text{(II)}$$

in welcher

Hal für Halogen, insbesondere Chlor oder Brom steht und

$R^1$ und n die oben angegebene Bedeutung haben,

mit Azolen der Formel

$$\text{H - Az} \qquad \text{(III)}$$

in welcher

Az die oben angegebene Bedeutung hat,

in Gegenwart eines Verdünnungsmittels und in Gegenwart eines Säurebindemittels umsetzt, und gegebenenfalls noch die so erhaltenen Keto-Derivate der Formel (I) nach bekannten Methoden in üblicher Weise reduziert, und gegebenenfalls noch an die Verbindungen der Formel (I) eine Säure oder ein Metallsalz addiert.

4) Fungizide Mittel, gekennzeichnet durch einen Gehalt an mindestens einem substituierten 1,1-Bis-azolylbutan-2-on oder -ol der Formel (I) in Ansprüchen 1 und 3.

5) Verfahren zur Bekämpfung von Pilzen, dadurch gekennzeichnet, daß man substituierte 1,1-Bis-azolyl-butan-2-one oder -ole der Formel (I) in Ansprüchen 1 und 3 auf Pilze oder ihren Lebensraum einwirken läßt.

6) Verwendung von substituierten 1,1-Bis-azolyl-butan-2-onen oder -olen der Formel (I) in Ansprüchen 1 und 3 als Pflanzenschutzmittel.

7) Verwendung von substituierten 1,1-Bis-azolyl-butan-2-onen oder -olen der Formel (I) in Ansprüchen 1 und 3 zur Bekämpfung von Pilzen.

8) Verfahren zur Herstellung von fungiziden Mitteln, dadurch gekennzeichnet, daß man substituierte 1,1-Bis-azolyl-butan-2-one oder -ole der Formel (I) in Ansprüchen 1 und 3 mit Streckmitteln und/oder oberflächenaktiven Mitteln vermischt.